(19) Europäisches Patentamt / European Patent Office / Office européen des brevets

(11) **EP 3 132 735 A1**

(12) **EUROPEAN PATENT APPLICATION**
published in accordance with Art. 153(4) EPC

(43) Date of publication:
22.02.2017 Bulletin 2017/08

(51) Int Cl.:
*A61B 1/00* (2006.01)     *G02B 13/04* (2006.01)
*G02B 13/18* (2006.01)     *G02B 23/26* (2006.01)

(21) Application number: 15779383.7

(22) Date of filing: 08.04.2015

(86) International application number:
PCT/JP2015/060931

(87) International publication number:
WO 2015/159770 (22.10.2015 Gazette 2015/42)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **14.04.2014 JP 2014082863**

(71) Applicant: **Olympus Corporation
Hachioji-shi
Tokyo 192-8507 (JP)**

(72) Inventor: **NAKAMURA, Minoru
Hachioji-shi
Tokyo 192-8507 (JP)**

(74) Representative: **Schicker, Silvia
Wuesthoff & Wuesthoff
Patentanwälte PartG mbB
Schweigerstraße 2
81541 München (DE)**

(54) **CAPSULE ENDOSCOPE**

(57) In the present invention, the viewing area is sufficiently enlarged, and an adequate level of illumination light is distributed in the enlarged viewing area. Provided is a capsule endoscope in which an objective lens unit (11) includes, at the extreme object side, a lens which is a meniscus lens having negative power and disposed with the convex surface thereof towards the object side, so that the convex surface thereof faces the exterior. An illumination unit (13) includes a light source and an illumination window member (19). The light source is disposed so as to surround the circumference of the lens. The illumination window member is formed in an approximately circular ring shape that surrounds the circumference of the lens and is positioned closer to the image side than an outermost diameter section in the convex surface of the first lens, through which a light flux passes. A light-blocking member that blocks the entry of illumination light into the objective lens unit is disposed between the illumination window member and the lens. Conditional expressions (1) and (2) below are satisfied:

$$-10 < f1/f < -4 \quad (1)$$

$$0.7 < d1/f < 2 \quad (2)$$

where f1 is the focal length of the lens, f is the focal length of the objective lens unit, and d1 is the distance between the lens and another lens disposed adjacent to the image side of the lens.

# FIG. 1

**Description**

{Technical Field}

The present invention relates to capsule endoscopes.

{Background Art}

In the related art, there are known capsule-type endoscopes that are swallowed by a subject and that capture images of the inside of the subject's body while moving inside the subject's body (for example, see Patent Literature 1 and Patent Literature 2).
It is difficult to control the position and orientation of this type of capsule endoscope inside the patient's body, and therefore, it is preferable that the viewing area of the image-acquisition optical system in the capsule endoscope be made as wide as possible so that as large an area as possible can be acquired even though the capsule endoscope does not change its orientation.

{Citation List}

{Patent Literature}

  {PTL 1} Japanese Unexamined Patent Application, Publication No. 2004-357933
  {PTL 2} Japanese Unexamined Patent Application, Publication No. 2008-43626

{Summary of Invention}

{Technical Problem}

However, in the capsule endoscope in the aforementioned Patent Literature 1, the power of the observation optical system is weak, thus narrowing the viewing area, and the illumination optical system also only illuminates the forward direction. In the capsule endoscope in Patent Literature 2, although the power of a cover of the observation optical system is high, thus making the viewing area comparatively wide, the viewing area cannot be said to be sufficiently large since the gap between the cover and a lens adjacent to the cover is large. In addition, even though a light emitting element in the illumination optical system protrudes outward from the case, and an illumination window also serves as part of the cover, there is no light-blocking member that shields the observation optical system from the illumination light. Therefore, unwanted light such as flare gets into the observation optical system, and there is a risk of degradation of the obtained image.
The present invention has been conceived in light of the circumstances described above, and an object thereof is to sufficiently enlarge the viewing area and also to distribute a sufficient amount of illumination light in the enlarged viewing area.

{Solution to Problem}

In order to realize the above objects, the present invention provides the following solutions.
A first aspect of the present invention is a capsule endoscope including a case, and an objective lens unit, an image acquisition device, and an illumination unit accommodated in the case; wherein the first lens, which is disposed on the extreme object side of the objective lens unit, is a meniscus lens having negative power and with a convex surface thereof facing the object side and is disposed so that at least the convex surface thereof faces the exterior of the case; the illumination unit includes a light source and an illumination window member that faces the exterior of the case and guides illumination light emitted from the light source to the exterior; the light source is disposed so as to surround the circumference of the first lens; the illumination window member is formed in an approximately circular ring shape that surrounds the circumference of the first lens and is positioned closer to the image side than an outermost diameter section of the convex surface of the first lens through which a light flux passes; a light-blocking member that blocks the entry of illumination light into the objective lens unit is disposed between the illumination window member and the first lens; and conditional expressions (1) and (2) below are satisfied

$$-10 < f1/f < -4 \quad (1)$$

$$0.7 < d1/f < 2 \quad (2)$$

where f1 is the focal length of the first lens, f is the focal length of the objective lens unit, and d1 is the distance between the first lens and a lens disposed adjacent to the image side of the first lens.

According to this aspect, with the object-side surface of the first lens assumed to be the convex surface, negative distortion becomes stronger, and from a paraxial calculation in which the powers of components of the first objective lens unit except for the first lens are combined, the power of the first lens is set to be negative, the lenses are configured so as to satisfy conditional expressions (1) and (2). By doing so, it is possible to ensure a viewing angle of 180° or more, which enables rearward observation.

If the lower limit of conditional expression (1) is exceeded, due to the fact that the power of the first lens becomes weaker, in order to reduce the overall focal length so as to ensure a sufficient viewing angle, from paraxial calculations, the overall length of the objective lens unit increases since it is necessary to increase the spacing between components other than the first lens. If the upper limit of conditional expression (1) is exceeded, the power of the first lens and the curvature of the surface on the image-plane side of the first lens become stronger, and it becomes difficult to ensure, on the surface at the image side of the first lens, a region through which the required light flux passes, and therefore, a viewing angle of 180 degrees or more becomes impossible, and aberrations deteriorate.

If the lower limit of conditional expression (2) is exceeded, the power of the first lens becomes too strong, degrading the performance, and if the upper limit is exceeded, the overall length increases, and the outer diameter becomes large.

In addition, by disposing, around the circumference of the first lens, the illumination window formed in an approximately circular ring shape that surrounds the first lens, it is possible to ensure a suitable light distribution and intensity for covering a viewing angle of 180° or more.

Since the light-blocking member that blocks the entry of illumination light into the objective lens unit disposed between the illumination window member and the first lens, it is possible to prevent unwanted light such as direct illumination light or flare reflected at a surface of the case or the like from entering the objective lens.

It is preferable to dispose the convex surface, which serves as the object-side surface of the first lens, so as to face the exterior of the case, and by doing so, the convex surface of the first lens functions as a portion of the case, which is the boundary with the exterior, and therefore, it is possible to reduce the overall length of the capsule endoscope.

In the above-described aspect, conditional expression (3) below may be satisfied:

$$1.1 < fm/f \le 1.3 \quad (3)$$

where fm is the focal length of components of the objective lens excluding the first lens.

By doing so, the number of lenses can be suppressed, and manufacturing costs can be controlled. If the lower limit of conditional expression (3) is exceeded, the power of the first lens becomes weaker, and therefore, the overall length of the objective lens unit increases. If the upper limit of conditional expression (3) is exceeded, the aberrations become worse, and the power of the first lens becomes stronger, and accordingly, the power of the components in the objective lens unit except for the first lens also becomes stronger. Because of this, there are some disadvantages, such as the need to increase the number of lenses to correct the aberrations, an increase in the overall length, and a rise in costs.

In the above-described aspect, a front surface shape as viewed from the object side of the light-blocking member may be a substantially rectangular shape that matches the outer shape of the image acquisition device.

By doing so, the projection area of the illumination window member in the forward direction can be enlarged, and therefore, the light distribution in the forward direction can be further improved.

In the above-described aspect, the light-blocking member may be formed as a single unit together with a lens frame that holds the objective lens unit.

By doing so, manufacturing costs can be reduced, and assembly of the objective lens unit, and by extension, the capsule endoscope, is facilitated. In addition, it is possible to further improve the blocking of unwanted light on the objective lens unit.

{Advantageous Effects of Invention}

The present invention affords an advantage in that it is possible to sufficiently enlarge the viewing area and to distribute a sufficient amount of illumination light in the enlarged viewing area.

{Brief Description of Drawings}

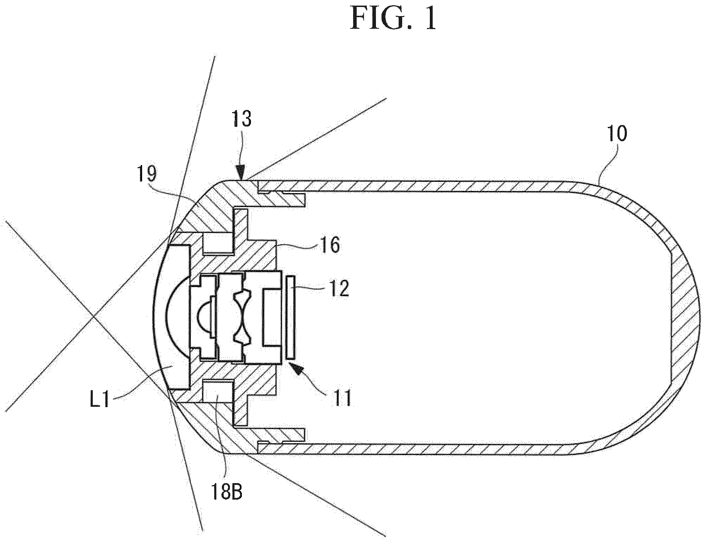

{Fig. 1}
Fig. 1 is a cross-sectional view showing the overall configuration of a capsule endoscope according to a first embodiment of the present invention.
{Fig. 2}
Fig. 2 is a cross-sectional view showing the overall configuration of an objective optical system used in the capsule endoscope according to the first embodiment of the present invention.
{Fig. 3}
Fig. 3 is a cross-sectional view showing the overall configuration of a lens holding frame used in the capsule endoscope according to the first embodiment of the present invention.
{Fig. 4}
Fig. 4 is a front view showing the overall configuration of a light source unit used in the capsule endoscope according to the first embodiment of the present invention.
{Fig. 5}
Fig. 5 is a cross-sectional view showing the overall configuration of an illumination window member used in the capsule endoscope according to the first embodiment of the present invention.
{Fig. 6}
Fig. 6 is a cross-sectional view showing the overall configuration of an illumination window member used in the capsule endoscope according to another example of the first embodiment of the present invention.
{Fig. 7}
Fig. 7 is a cross-sectional view showing the overall configuration of an illumination window member used in a capsule endoscope according to a modification of the first embodiment of the present invention.
{Fig. 8}
Fig. 8 is a cross-sectional view showing the overall configuration of a lens holding frame used in the capsule endoscope according to the modification of the first embodiment of the present invention.
{Fig. 9}
Fig. 9 is a cross-sectional view showing the overall configuration of a capsule endoscope according to a second embodiment of the present invention.
{Fig. 10}
Fig. 10 is a cross-sectional view showing the overall configuration of an objective optical system used in the capsule endoscope according to the second embodiment of the present invention.
{Fig. 11}
Fig. 11 is a cross-sectional view showing the overall configuration of an example of a capsule endoscope that can observe in two directions.
{Fig. 12}
Fig. 12 is a reference perspective view showing the concept of an example of a capsule endoscope that can observe in six directions.
{Fig. 13}
Fig. 13 is a cross-sectional view showing the overall configuration of the example of the capsule endoscope in Fig. 12, which can observe in six directions.

{Description of Embodiments}

First Embodiment

A capsule endoscope according to a first embodiment of the present invention will be described below with reference to the drawings.
Fig. 1 is a cross-sectional view showing the overall configuration of a capsule endoscope according to this embodiment. The capsule endoscope includes a case 10, an objective lens unit 11 that is accommodated in the case 10 and that forms an image of a subject, an image acquisition device 12 that acquires the image of the subject formed by the objective lens unit 11, and an illumination unit 13 that irradiates the subject with illumination light.
The objective lens unit 11 includes an objective optical system 15 formed of a plurality of lenses and a lens holding frame 16 that holds the objective optical system.

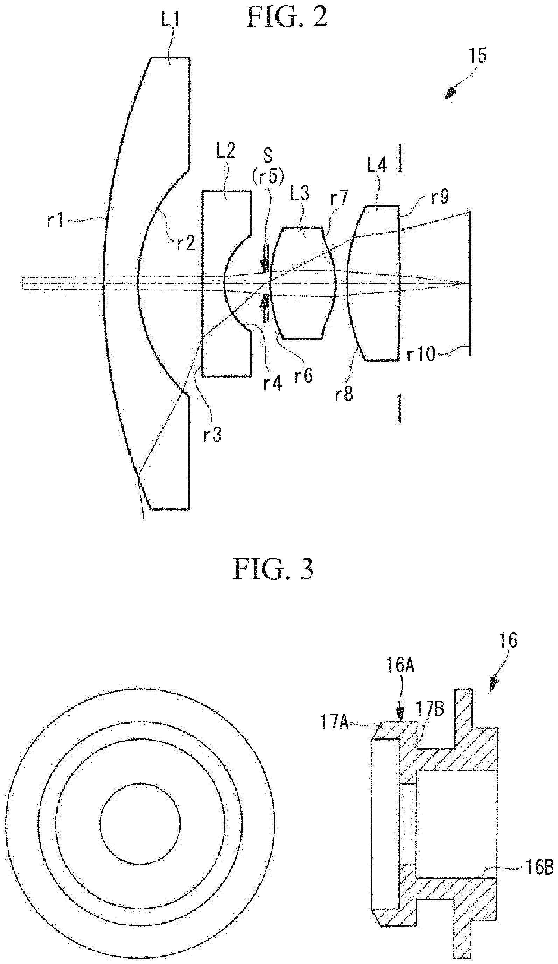

As shown in Fig. 2, the objective optical system 15 is formed of a first lens L1, a second lens L2, an aperture stop S, a third lens L3, and a fourth lens L4, in this order from the object side, and the first lens L1, which is disposed at the extreme object side of the objective optical system 15, is a meniscus lens in which the convex surface faces the object side and which has negative power.

The objective optical system 15 is configured so as to satisfy conditional expressions (1) and (2) below:

$$-10 < f1/f < -4 \quad (1)$$

$$0.7 < d1/f < 2 \quad (2)$$

where f1 is the focal length of the first lens, f is the focal length of the objective lens unit, and d1 is the distance between the first lens and the lens disposed adjacent to the image side of the first lens.

If the lower limit of conditional expression (1) is exceeded, due to the fact that the power of the first lens becomes weaker, in order to reduce the overall focal length so as to ensure a sufficient viewing angle, from paraxial calculations, the overall length of the objective lens unit increases since it is necessary to increase the spacing between components other than the first lens. If the upper limit of conditional expression (1) is exceeded, the power of the first lens and the curvature of the surface on the image-plane side of the first lens become stronger, and it becomes difficult to ensure, on the surface at the image side of the first lens, a region through which the required light flux passes, and therefore, a viewing angle of 180 degrees or more becomes impossible, and aberrations deteriorate.

If the lower limit of conditional expression (2) is exceeded, the power of the first lens becomes too strong, degrading the performance, and if the upper limit is exceeded, the overall length increases, and the outer diameter becomes large.

In addition, conditional expression (3) below is satisfied:

$$1.1 < fm/f \le 1.3 \quad (3)$$

where fm is the focal length of the components of the objective lens, excluding the first lens.

If the lower limit of conditional expression (3) is exceeded, the power of the first lens becomes weaker, and therefore, the overall length of the objective lens unit increases. If the upper limit of conditional expression (3) is exceeded, the aberrations become worse, and the power of the first lens becomes stronger, and accordingly, the power of the components in the objective lens unit except for the first lens also becomes stronger. Because of this, there are some disadvantages, such as the need to increase the number of lenses to correct the aberrations, an increase in the overall length, and a rise in costs.

As shown in Fig. 3, the lens holding frame 16 has a first holding section 16A that is formed in a ring shape when viewed from the front and that holds the first lens L1, and a second holding section 16B that holds the second lens L2, the third lens L3, and the fourth lens L4. The first holding section 16A has a side-surface holding section 17A that holds a side surface of the first lens L1 and a flat-surface holding section 17B that holds the surface at the image plane side of the first lens L1.

When held in the first holding section 16A, the first lens is disposed so that the convex surface, which is the surface that faces the object side of the first lens L1, faces the outside of the case 10, so that the convex surface of the first lens L1 functions as a portion of the case.

The second holding section 16B holds the side surfaces of the second lens L2, the third lens L3, and the fourth lens L4 and is configured so that the second lens L2, the third lens L3, and the fourth lens L4 are accommodated in a space formed by the first lens L1 and the second holding section 16B.

By forming the lens holding frame 16 of a light blocking material, the lens holding frame functions as a light-blocking member. In other words, a light-blocking member that blocks the entry of the illumination light into the objective lens unit 11 is disposed between a unit 18 described later and the first lens L1. Therefore, the entry of illumination light from the illumination unit 13 disposed outside the lens holding frame 16 into the objective optical system 15 accommodated in the lens holding frame 16 can be blocked.

The illumination unit 13 is provided with the light source unit 18 and an illumination window member 19 that guides the illumination light emitted from the light source unit 18 to the outside.

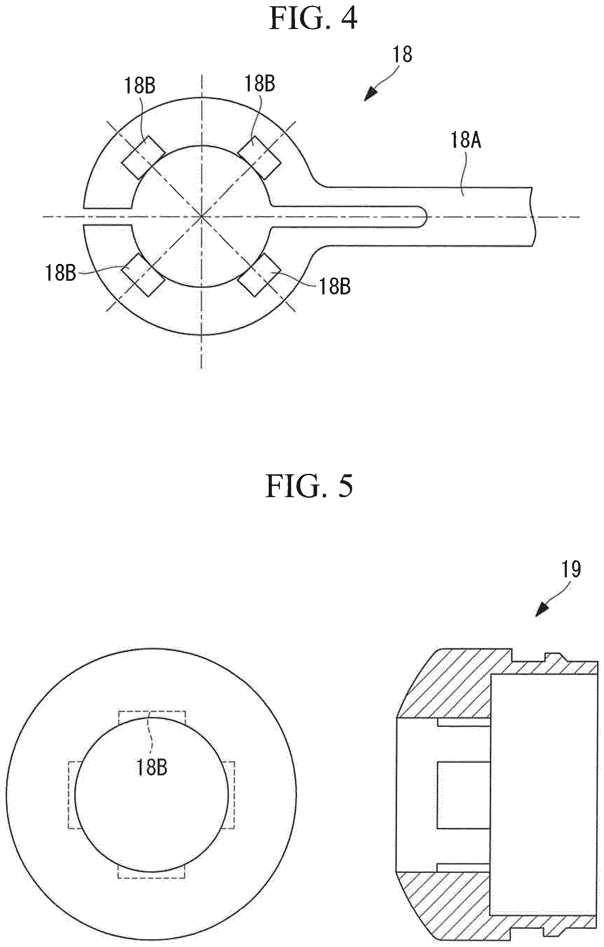

As shown in Fig. 4, the light source unit 18 is provided with a flexible substrate 18A formed in an approximately circular ring shape that surrounds the circumference of the first lens L1 when viewed from the front and a plurality of LEDs 18B disposed uniformly on this flexible substrate. Fig. 4 shows an example in which four LEDs are disposed at 90° intervals, and are disposed so as to surround the first lens L1.

As shown in Fig. 5, the illumination window member 19 is formed in an approximately circular ring shape that surrounds the circumference of the first lens when viewed from the front, is fitted on the end of the case 10, and is formed so that the light source unit 18 and the lens holding frame 16 are accommodated at the inner side of the illumination window member 19, so as to be positioned closer to the image side than the outermost diameter section of the convex surface of the first lens L1 through which the light flux passes. The outer surface of the illumination window member 19 faces outside the case 10, and the convex surface of the first lens, the end surface of the side-surface holding section 17A of the lens holding frame, which holds the first lens, and the outer surface of the illumination window member are disposed to form a single surface, so as to function as a portion of the case.

According to this embodiment, with the object-side surface of the first lens assumed to be the convex surface, negative distortion becomes stronger, and from a paraxial calculation in which the powers of components of the objective lens unit other than the first lens are combined, the lenses are configured so that, with the power of the first lens assumed to be negative, conditional expressions (1) and conditional expression (2) are satisfied. By doing so, a viewing angle of 180° and higher, with which it is possible to perform rearward observation, can be ensured.

In addition, by disposing the illumination window, which is formed in an approximately circular ring shape that surrounds the first lens, around the circumference of the first lens, it is possible to ensure a suitable light distribution and intensity for covering a viewing angle of 180° or more.

By forming the lens holding frame 16 of a light blocking material, the light-blocking member that blocks the entry of illumination light into the objective lens unit is disposed between the illumination window member and the first lens, and it is possible to prevent unwanted light such as direct illumination light or flare reflected at a surface of the case or the like from entering the objective lens.

In addition, by disposing the convex surface of the first lens so as to face the exterior of the case, the convex surface of the first lens functions as a portion of the case, which is the boundary with the exterior, and therefore, it is possible to reduce the overall length of the capsule endoscope.

Modification 1

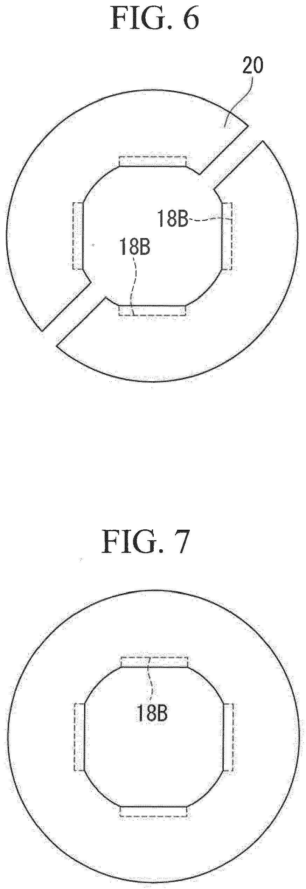

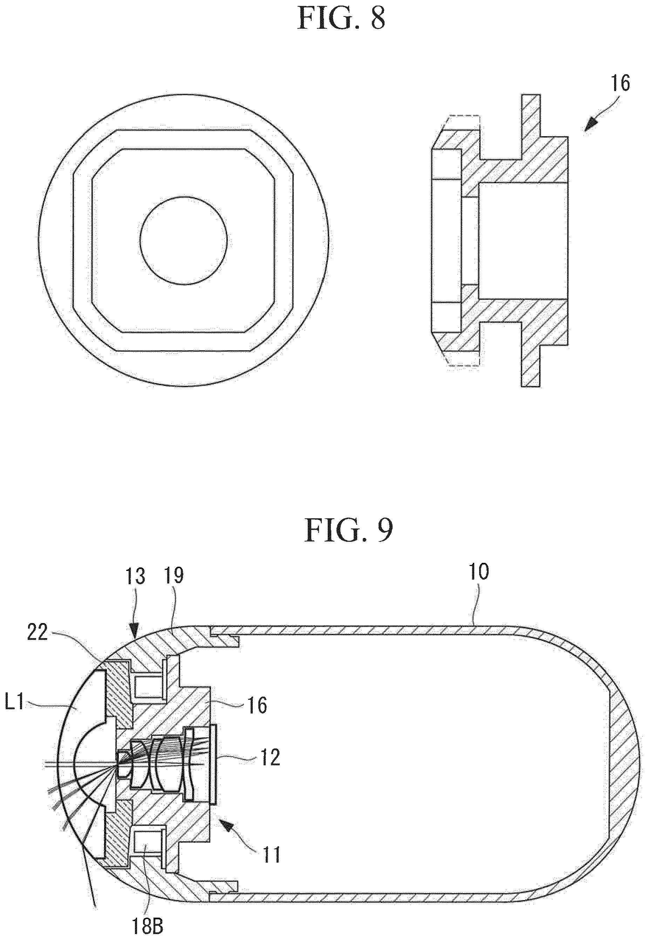

In the first embodiment described above, the lens holding frame 16 and the illumination window member 19 are formed to have approximately circular ring shapes in which the inner side and the outer side are both circular shapes so as to be able to hold the first lens, which has a circular shape when viewed from the front; however, it is not necessarily limited thereto. Specifically, due to the fact that the image-acquisition device that forms an image of the subject has a rectangular shape, the first lens can be made to have a rectangular shape that matches the long side and short side of the image acquisition device to the extent that the light flux required for forming an image is not blocked, and the inner shapes of the lens holding frame 16 and the illumination window member 19 can be made to have approximately rectangular shapes so as to match the shape of this first lens (see Fig. 7 and Fig. 8).

By doing so, the projection area of the illumination window member can be enlarged, and the light distribution in the forward direction can be further improved.

In the above-described first embodiment and the first modification thereof, the illumination window member 19 is formed in an approximately circular shape that surrounds the circumference of the first lens when viewed from the front; however, it is not necessarily limited thereto. For example, as shown in Fig. 6, a configuration in which a plurality of circular arc-shaped illumination window members 20 are disposed in the form of an approximately circular ring is possible.

Examples

Lens data for the objective optical systems according to Example 1 and Example 2 of the aforementioned first embodiment and modification 1 thereof are shown below. In the lens data below, r indicates the radius of curvature (unit, mm), d indicates the inter-surface distance (mm), Nd indicates the refractive index at the d-line, and Vd indicates the Abbe number at the d-line. Aspheric surfaces are indicated by a * on the surface numbers in the lens data, and the paraxial radius of curvature r, the conic constant K, and the aspheric coefficients Ai (i = 2, 4, 6, 8, 10) of the aspheric surface shape expressed by the following equation are shown in the aspheric surface data. Note that, in the following equation, the optical axis direction is z, and the direction perpendicular to the optical axis is y.

$$z = (y^2/r) \; / \; [1 + \{1 - (1+K)(y/r)^2\}^{1/2}]$$

$$+ A2y^2 + A4y^4 + \ldots + A8y^8 + A10y^{10}$$

Example 1

Lens data for the objective optical system in Example 1 according to the above-mentioned first embodiment and modification 1 thereof are shown below.

Lens Data

| Surface number | r | d | Ne | Vd |
|---|---|---|---|---|
| Object plane | ∞ | ∞ | 1. | |
| 1 | 9.5885 | 0.6480 | 1.53296 | 55.69 |
| 2 | 2.5855 | 1.2138 | 1. | |
| 3 | 51.8397 | 0.3888 | 1.53296 | 55.69 |
| 4 | 0.9694 | 0.7776 | 1. | |
| 5 Aperture stop | ∞ | 0.0959 | 1. | |
| 6 | 2.0723 | 1.2182 | 1.53296 | 55.69 |
| 7* | -1.2571 | 0.2074 | 1. | |
| 8 | 2.8395 | 1.0368 | 1.53296 | 55.69 |
| 9 | -21.5783 | | 1.3396 | 1. |
| 10(image plane) | ∞ | | | |

Aspheric surface data
Surface 7

r=-1.2571

K=-1.8390

A2=0.0

A4=4.0166e-03

A6=4.9015e-02

A8=8.1832e-02

A10=-3.0745e-02

Viewing angle (2ω) 200.8°

| Maximum image height | 1.264 |
|---|---|
| Fno. | 4.625 |
| Focal length | 1.000 |

f1/f: -6.86
d1/f: 1.21
fm/f: 1.22

Example 2

Lens data for the objective optical system in Example 2 according to the above-mentioned first embodiment and modification 1 thereof are shown below.

Lens Data

| Surface number | r | d | Ne | Vd |
|---|---|---|---|---|
| Object plane | ∞ | ∞ | 1. | |
| 1 | 7.4466 | 0.7195 | 1.53296 | 55.69 |
| 2 | 2.0518 | 1.1231 | 1. | |
| 3 | -62.9078 | 0.4317 | 1.53296 | 55.69 |
| 4 | 0.9371 | 0.9144 | 1. | |
| 5 Aperture stop | ∞ | 0.1284 | 1. | |
| 6 | 1.8545 | 1.3528 | 1.53296 | 55.69 |
| 7* | -1.2470 | 0.2303 | 1. | |
| 8 | 4.9190 | 1.1513 | 1.53296 | 55.69 |
| 9 | -65.3777 | 1.4650 | 1. | |
| 10(image plane) | ∞ | | | |

Aspheric surface data
Surface 7

r=-1.2470
K=-1.9262
A2=0.0
A4=-8.1063e-03
A6=2.2254e-02
A8=1.1724e-01
A10=-1.7269e-02

Viewing angle (2ω) 201.4°
Maximum image height 1.403
Fno. 4.581
Focal length 1.000

f1/f: -5.57
d1/f: 1.12
fm/f: 1.26

Second Embodiment

Next, a second embodiment of the present invention will be described. In this embodiment, configurations that are identical to those in the first embodiment described above are assigned the same reference signs, and descriptions thereof are omitted. The differences between this embodiment and the capsule endoscope according to the first embodiment and modification 1 thereof are as follows.

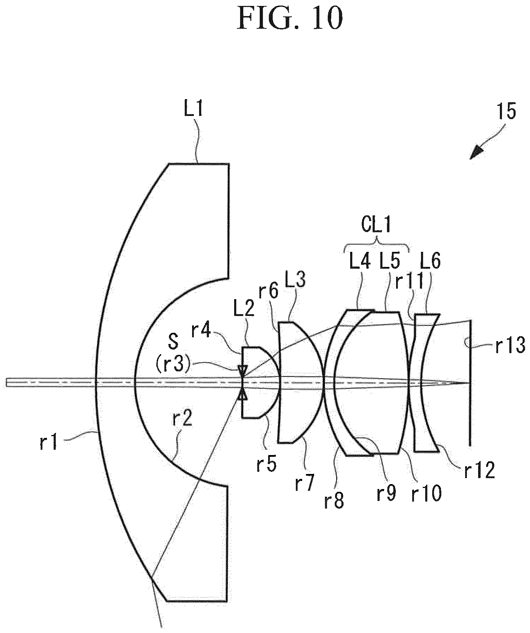

Specifically, as shown in Figs. 9 and 10, the objective optical system is formed of a first lens L1, an aperture stop S, a second lens L2, a third lens L3, a fourth lens L4, a fifth lens L5, and a sixth lens L6, in this order from the object side, where the fourth lens and the fifth lens are combined to form a combined lens CL1. Chromatic aberration of magnification can be corrected more effectively by using the combined lens CL1, field curvature can be corrected by using a meniscus lens at the sixth lens L6, and in addition, by using aspheric surfaces, aberrations can be corrected more effectively. Accordingly, an objective optical system that can also be used for a high-precision image acquisition device becomes possible.

In addition, the lens holding frame 16 and the light-blocking member 22 are separate, independent structures rather than being combined in a single unit.

The lens data of the objective optical system according to the second embodiment described above is as follows.

Lens data

| Surface number | r | d | Ne | Vd |
|---|---|---|---|---|
| Object | ∞ | ∞ | 1. | |
| 1 | 7.0056 | 0.6369 | 1.58959 | 30.00 |
| 2 | 1.9106 | 1.8469 | 1. | |
| 3 Aperture stop | ∞ | 0.0319 | 1. | |
| 4 | -0.7192 | 0.5581 | 1.53336 | 56.00 |
| 5* | -0.6479 | 0.0274 | 1. | |
| 6 | -10.8727 | 0.6858 | 1.53336 | 56.00 |
| 7* | -3.4092 | 0.0274 | 1. | |
| 8 | 2.5028 | 0.1824 | 1.93429 | 18.90 |
| 9 | 1.5989 | 1.2252 | 1.73234 | 54.68 |
| 10 | -4.7961 | 0.0274 | 1. | |
| 11* | 3.8546 | 0.1824 | 1.53336 | 56.00 |
| 12 | 2.5989 | 0.8211 | 1. | |
| 13 (image plane) | ∞ | | | |

Aspheric surface data
Surface 5
r=-0.6479
K=-0.5730
A2=0.0
A4=1.6518e-01
A6=-4.9711e-02

Surface 7

r=-3.4092
K=0.3236
A2=0.0
A4=-2.7518e-01
A6=8.9225e-02
A8=-5.3971e-02

Surface 11

r=3.8546
K=-0.7326

A2=0.0
A4=-5.8668e-02
A6=-1.5556e-02
A8=9.0343e-03

Viewing angle (2ω) 200.1°
Maximum image height 1.14

Fno. 7.591

Focal length 1.000

f1/f: -5.57
d1/f: 1.12
fm/f: 1.26

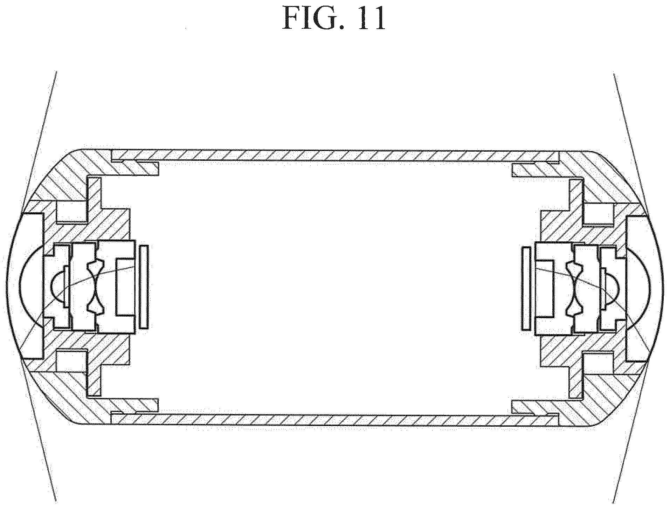

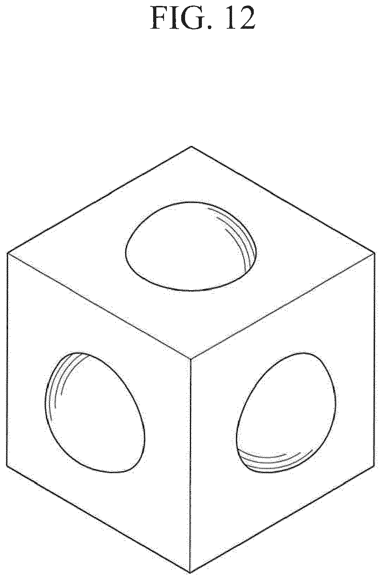

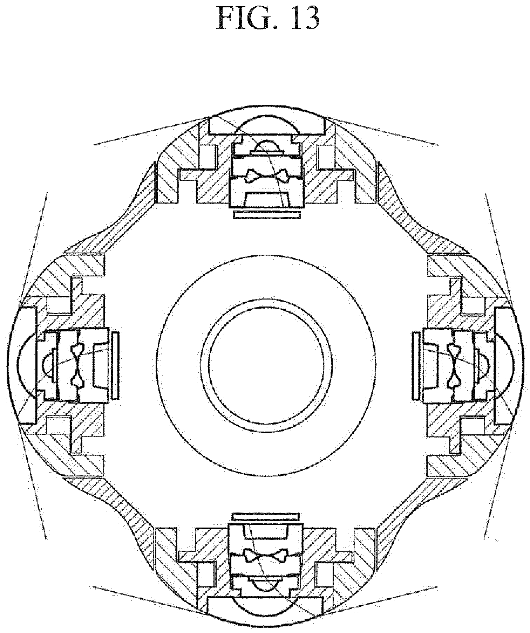

In the above embodiments, although a configuration in which the objective optical system and so forth are provided only in one direction in the capsule endoscope has been described, it is not limited thereto; for example, as shown in Fig. 11, a configuration that enables observation in two directions, namely, the forward and rearward directions, of the capsule endoscope is also possible, and as shown in Fig. 12 and Fig. 13, a configuration that enables observation in six directions by using a substantially cube shaped capsule endoscope is also possible.

{Reference Signs List}

| | |
|---|---|
| 10 | Case |
| 11 | Objective lens unit |
| 12 | Image acquisition device |
| 13 | Illumination unit |
| 15 | Objective optical system |
| 16 | Lens holding frame |
| 18 | Light source unit |
| 19, 20 | Illumination window member |
| 22 | Light-blocking member |
| L1 | First lens |
| L2 | Second lens |
| L3 | Third lens |
| L4 | Fourth lens |
| L5 | Fifth lens |
| L6 | Sixth lens |
| CL1 | Combined lens |

**Claims**

1. A capsule endoscope comprising a case, and an objective lens unit, an image acquisition device, and an illumination unit accommodated in the case;
   wherein the first lens, which is disposed on the extreme object side of the objective lens unit, is a meniscus lens having negative power and with a convex surface thereof facing the object side and is disposed so that at least the convex surface thereof faces the exterior of the case;
   the illumination unit includes a light source and an illumination window member that faces the exterior of the case and guides illumination light emitted from the light source to the exterior;

the light source is disposed so as to surround the circumference of the first lens;

the illumination window member is formed in an approximately circular ring shape that surrounds the circumference of the first lens and is positioned closer to the image side than an outermost diameter section of the convex surface of the first lens through which a light flux passes;

a light-blocking member that blocks the entry of illumination light into the objective lens unit is disposed between the illumination window member and the first lens; and conditional expressions (1) and (2) below are satisfied

$$-10 < f1/f < -4 \quad (1)$$

$$0.7 < d1/f < 2 \quad (2)$$

where f1 is the focal length of the first lens, f is the focal length of the objective lens unit, and d1 is the distance between the first lens and a lens disposed adjacent to the image side of the first lens.

2. A capsule endoscope according to claim 1, wherein conditional expression (3) below is satisfied:

$$1.1 < fm/f \leqq 1.3 \quad (3)$$

where fm is the focal length of components of the objective lens excluding the first lens.

3. A capsule endoscope according to claim 1 or claim 2, wherein a front surface shape of the light-blocking member as viewed from the object side is a substantially rectangular shape that matches the outer shape of the image acquisition device.

4. A capsule endoscope according to one of claims 1 to 3, wherein the light-blocking member is formed as a single unit together with a lens frame that holds the objective lens unit.

# FIG. 1

EP 3 132 735 A1

FIG. 2

FIG. 3

# FIG. 4

18B    18B

18

18A

18B    18B

# FIG. 5

19

18B

# FIG. 6

20

18B

18B

# FIG. 7

18B

# FIG. 8

# FIG. 9

# FIG. 10

FIG. 11

# FIG. 12

# FIG. 13

| **INTERNATIONAL SEARCH REPORT** | International application No. |
|---|---|
| | PCT/JP2015/060931 |

**A. CLASSIFICATION OF SUBJECT MATTER**
*A61B1/00*(2006.01)i, *G02B13/04*(2006.01)i, *G02B13/18*(2006.01)i, *G02B23/26* (2006.01)i

According to International Patent Classification (IPC) or to both national classification and IPC

**B. FIELDS SEARCHED**

Minimum documentation searched (classification system followed by classification symbols)
A61B1/00-1/32, G02B13/04, G02B13/18, G02B23/24-23/26

Documentation searched other than minimum documentation to the extent that such documents are included in the fields searched
Jitsuyo Shinan Koho          1922-1996   Jitsuyo Shinan Toroku Koho   1996-2015
Kokai Jitsuyo Shinan Koho    1971-2015   Toroku Jitsuyo Shinan Koho   1994-2015

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2005-80713 A  (Olympus Corp.),<br>31 March 2005 (31.03.2005),<br>paragraphs [0010] to [0039]; fig. 1 to 5<br>& US 2005/0124858 A1 | 1-4 |
| A | JP 2010-85484 A  (Kyocera Optec Co., Ltd.),<br>15 April 2010 (15.04.2010),<br>paragraphs [0039] to [0046]<br>(Family: none) | 1-4 |
| A | WO 2006/129472 A1  (Konica Minolta Medical &<br>Graphic, Inc.),<br>07 December 2006 (07.12.2006),<br>paragraphs [0031], [0065]; fig. 2<br>& JP 5003486 B2          & EP 1886618 A1 | 1-4 |

☒  Further documents are listed in the continuation of Box C.          ☐  See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 26 June 2015 (26.06.15) | 07 July 2015 (07.07.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

22

**INTERNATIONAL SEARCH REPORT**

| International application No. |
| --- |
| PCT/JP2015/060931 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 10-288742 A  (Olympus Optical Co., Ltd.), 27 October 1998 (27.10.1998), paragraphs [0009] to [0013]; fig. 1 to 5 (Family: none) | 1-4 |
| A | JP 7-140329 A  (Fujikura Ltd.), 02 June 1995 (02.06.1995), entire text; all drawings (Family: none) | 1-4 |

Form PCT/ISA/210 (continuation of second sheet) (July 2009)

**EP 3 132 735 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004357933 A
- JP 2008043626 A